(19) Europäisches Patentamt
European Patent Office
Office européen des brevets

(11) **EP 2 502 478 A1**

(12) **EUROPEAN PATENT APPLICATION**

(43) Date of publication:
**26.09.2012 Bulletin 2012/39**

(51) Int Cl.:
*A01C 3/02* (2006.01)     *B65D 88/16* (2006.01)
*F17B 1/26* (2006.01)

(21) Application number: **11159655.7**

(22) Date of filing: **24.03.2011**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Verwanger, Peter**
**8041 Graz (AT)**

(72) Inventors:
• **Verwanger, Peter**
**8041, Graz (AT)**
• **Verwanger, Irene**
**8041, Graz (AT)**

(74) Representative: **Dilg, Haeusler, Schindelmann Patentanwaltsgesellschaft mbH**
**Leonrodstrasse 58**
**80636 München (DE)**

(54) **Covering for a gas storage with an exterior membrane and a support structure**

(57)     The present invention relates to a covering for a gas storage (100). The covering comprises an exterior membrane (101) for at least partially enveloping a storage volume (Vs) of the storage (100), wherein the exterior membrane (101) is made of an elastomeric material, so that a shape and an area size of the exterior membrane (101) is adaptable to a pressure of the gaseous medium in the storage volume (Vs). The storage (100) further comprises a support structure mounted over the exterior membrane (101) in such a way that a) if a first pressure value in the storage volume (Vs) is reached, a first portion (111) of the exterior membrane (101) contacts the support structure in a force transmitting manner and a second portion (112) of the exterior membrane (101) is contact-free with the support structure, and b) if a critical pressure value storage volume (Vs) is reached, at least one of the support structure and the exterior membrane (101) fails, wherein if the critical pressure value in storage volume (Vs) is reached, the second portion (112) is expandable such that a contribution (Va) to the storage volume (Vs) of at least 10% in comparison to the volume of the storage volume (Vs) with the first pressure value is generated.

Fig. 1

## Description

Field of the invention

The present invention relates to a covering for a gas storage and to the gas storage. Moreover, present invention relates to a method of manufacturing a covering for a gas storage.

Background of the invention

Gas storages for storing gaseous medium, such as natural gas or biogas, comprise a covering with a flexible exterior membrane, which at least partially envelopes a storage volume of a gaseous medium that is stored in the gas storage. The exterior membrane is mounted to an edge of a sidewall of the gas storage such that the exterior membrane and the gas storage form a gas tight storage volume. The exterior membrane is inflated by the gas pressure of the gaseous medium in the storage volume. If the exterior membrane is completely inflated, the exterior membrane is inflated and thus stable. If the gas pressure in the storage volume is reduced by draining off stored gas, the exterior membrane tightens and lowers its position.

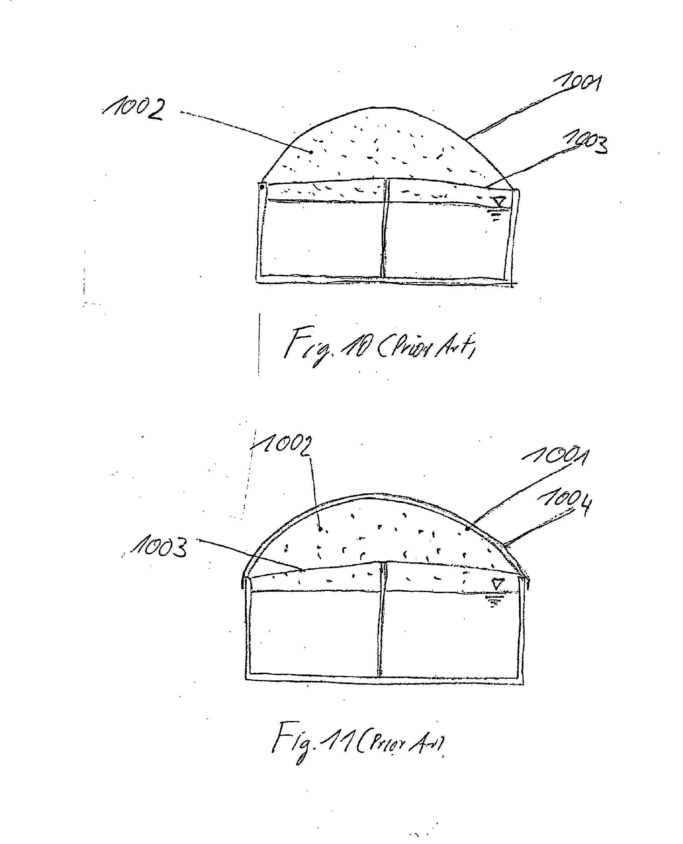

**Fig. 10** shows a gas storage with the exterior membrane 1001. The exterior membrane 1001 is mounted to an edge of the sidewall of the gas storage and envelopes the storage volume 1002 for storing gas. By the gas pressure inside the storage volume 1002, the exterior membrane 1001 is stretched until a balloon like shape is formed. The storage volume 1002 is thus changeable such that further gas is feedable into and drainable off the storage volume 1002. The lower position of the exterior membrane 1001 is limited by an inner support structure 1003, which may be formed out of wood, for example. By the inner support structure 1003, a contact of the exterior membrane 1001 with installations inside the gas storage or a contact with the stored substrate inside the biogas storage is prevented.

In order to increase the stability of the exterior membrane 1001 against exterior loads, such as wind or snow, a sufficiently high gas pressure in the storage volume 1002 is required.

If the gas storage comprises a large diameter, only a small gas pressure may be applied, because the stretched elongation of the exterior membrane 1001 would reach critical dimension and the exterior membrane 1001 would crack. Hence, in large gas storages, the elongation of the exterior membrane 1001 is limited by a close meshed mesh which is attached over the entire outer surface of the exterior membrane 1001.

**Fig. 11** shows an embodiment with a close meshed mesh 1004. The mesh 1004 acts as a barrier which prevents the exterior membrane 1001 from further elongation. The exterior membrane 1001 is not longer stretchable until the exterior membrane is nestled at the mesh 1004, even if the gas pressure increases further.

Hence, a higher gas pressure may be applied. Hence, the stability of the exterior membrane 1001 against wind loads and snow loads is increased. Summarizing, for a proper stability the exterior membrane 1001 should lie against the support structure and the gas pressure should be high. By this arrangement, where the close meshed mesh which is attached over the entire outer surface of the exterior membrane 1001, a variable gas storage capacity is not longer possible, because the exterior membrane 1001 is limited in its elongation. Excessive gas may not be stored inside the storage volume 1004. A draining off gas out of the gas storage is not possible, because the exterior membrane 1001 would lose the stabilizing contact to the mesh 1004 due to the pressure decrease.

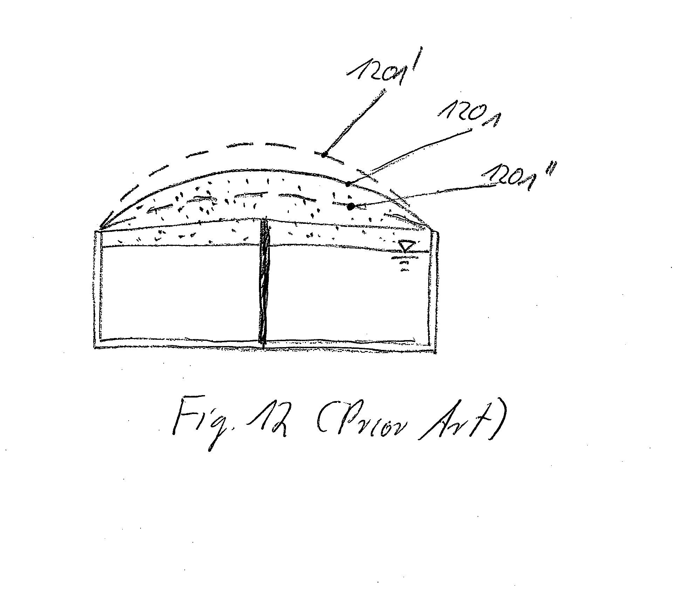

**Fig. 12** shows a conventional gas storage with an exterior membrane 1201 which is shown in three stretch positions. The exterior membrane 1201' is further stretched and elongated due to a higher pressure inside the gas storage in comparison to the lower position of exterior membrane 1201'', where gas is drained off the gas storage.

The designs without the mesh and with the entirely covering mesh have the following disadvantages. Without the mesh, the exterior membrane covering large areas has low wind stability, because the large sized exterior membranes can only withstand low gas pressure. By covering the complete area of the exterior membrane with the mesh, the adaptable storage function of the stretchable exterior membrane is not longer available, because the mesh prevents a further elongation and thus a further volume increase of the storage volume. Furthermore, in a lower position of the exterior membrane in the gas storage, the exterior membrane is not elongated and thus has a lower tension, which leads to a lower wind stability. Hence, there is a risk that the exterior membrane begins to fluttering.

Object and summary of the invention

It may be an object of the present invention to provide a robust covering for a gas storage independent of the gas filling level in the gas storage.

This object may be solved by a covering for a gas storage and by a method of manufacturing a covering for a gas storage according to the independent claims.

According to a first aspect of the present invention, a covering for a gas storage is presented. The covering comprises an exterior membrane for at least partially enveloping a storage volume of the storage. The exterior membrane is made of an elastomeric material, so that the shape and an area size of the exterior membrane is adaptable to a pressure of the gaseous medium in the storage volume. Moreover, the covering comprises a support structure mounted over the exterior membrane in such a way that a) if a first pressure value in the storage volume is reached, a first portion of the exterior membrane contacts the support structure in a force transmitting manner and a second portion of the exterior membrane is contact-free with the support structure, and b) if a critical pressure value storage volume is reached, at least one of the support structure and the exterior membrane fails, wherein if the critical pressure value in the storage volume is reached, the second portion is expandable such that a contribution (e.g. generated by the expansion of the second portion) to the storage volume of at least 10% in comparison to the volume of the storage volume with the first pressure value is generated.

According to a further aspect of the present invention, a method of manufacturing a covering for a gas storage is presented. A storage volume of the storage for the gaseous medium is at least partially enveloped with an exterior membrane, wherein the exterior membrane is made of an elastic material, so that a shape and an area size of the exterior membrane is adaptable to a pressure of the gaseous medium in the storage volume. A support structure is mounted over the exterior membrane in such a way a) if a first pressure value in the storage volume is reached, a first portion of the exterior membrane contacts the support structure in a force transmitting manner and a second portion of the exterior membrane is contact-free with the support structure, and b) if a critical pressure value storage volume is reached, at least one of the support structure and the exterior membrane fails, wherein if the critical pressure value in storage volume is reached, the second portion is expandable such that a contribution to the storage volume of at least 10% in comparison to the volume of the storage volume with the first pressure value is generated.

The storage for a gaseous medium may comprise any desired tubular shape, for example with a circular, oval, rectangular or polygonal ground area. An open top side of the storage is covered by the above-described exterior membrane, so that within the interior of the storage the storage volume for the gaseous medium is provided.

The above described covering is usable for covering a biogas storage (fermenter) or a conventional industrial gas storage. Moreover, covering is usable for covering a slurry storage. Due to a volume expansion during daytime and a volume reduction during night-time of the (slurry) gas in the storage volume, the exterior membrane may compensate the volume change by its stretching characteristic. A gas exchange with the environment is not necessary. Hence, bad smell of a slurry storage may be prevented.

The gaseous medium comprises components of industrial gas, such as natural gas or biogas, and components of support gas, such as air or inert gas.

Moreover, the storage device may be a biogas storage, so that additionally to the gaseous medium biomass may be stored within the storage. In this case, the volume for the gaseous medium may be the volume that is enveloped by the biomass, the membrane and in some cases the sidewall of the storage.

The support structure may comprise a plurality of elongated restraining elements, such as cables, belts or ropes that are fixed to the ground plate or to a sidewall of the storage. The support structure may be formed with flabby and flexible elements.

The first portion defines the portion of the exterior membrane that is substantially in contact the support structure in a force transmitting manner, so that a force may be transferred between the first portion and the support structure. Hence, when contacting with the first portion to the support structure in the force transmitting manner, the exterior membrane is restrained.

In particular, the contacting in the force transmitting manner between the first portion and the support structure is generated, if the first pressure value in the storage is exceeded. If the pressure in the storage volume is below the first pressure value, the support structure may lay onto the exterior membrane without being tensioned by the exterior membrane. If the first pressure value is exceeded, the first portion presses against the support structure, such that the support structure is expanded and tensioned by the exterior membrane. If the first portion of the exterior membrane presses against the support structure and if the support structure is thereby tensioned, a force may be transferred from the exterior membrane to the support structure for supporting the exterior membrane. In particular, if the exterior membrane is expanded and the first pressure value in the storage volume is exceeded, the support structure is sufficiently tensioned by the first portion of the exterior membrane, so that in this condition a contact with a force transmission is generated and the first portion is reinforced by the support structure.

The second portion is contact-free with the support structure. Moreover, the second portion is designed in such a way that if the pressure of the storage volume exceeds the first pressure value, the second portion of the exterior membrane further expands and is stretched, so that the area size of the second portion is increased. In other words, the second portion may balloon and form a balloon like shape if the pressure of the storage volume exceeds the first pressure value. Hence, the second portion envelops (i.e. with its balloon like shape) a contribution of the storage volume, wherein the contribution of the storage volume increases with the further stretching of the second portion. In particular, the contribution of the storage volume generated by the second portion is at least 10% of the total storage volume under the critical pressure value in the storage volume. In particular, a projected area of the exterior membrane and/or the support structure, which projected area is formed by a projection of the exterior membrane and/or the support structure onto the ground along a vertical direction, may have an area size of the second portion which is larger than approximately 20 $m^2$, 30 $m^2$, 50 $m^2$ or 100 $m^2$ (square meter).

The achievable volume of the contribution to the storage volume by the second portion may be defined by a value for the achievable volume under the critical pressure value at which the exterior membrane and/or the support structure fails and breaks. Moreover, the achievable volume of the contribution to the storage volume by the second portion may be defined by a value for the achievable volume under the critical pressure value at which the exterior membrane just have reversible and almost elastic properties, so that the exterior membrane can still shrink approximately to its initial shape and size. In particular, the exterior membrane according to the present invention is formed in such a way that the exterior membrane may form before breaking and failing a contribution of at least 10% of the total storage volume under the critical pressure in the storage volume.

The storage volume denotes the gas compartment which is generated between the exterior membrane and a virtual surface (plane) which connects an upper storage edge of the gas storage, to which upper storage edge the exterior membrane is mounted. The storage volume describes the volume enveloped by the exterior membrane and the virtual surface at the upper edge of the gas storage, in particular the upper edge of the walls of the gas storage. In other words, the storage volume is also described by the virtual surface (plane) which connects the edge of the exterior membrane, with which edge the exterior membrane is connected to the upper edge of (the wall of) the gas storage. By determining the surface shape (surface integral) of the exterior membrane and the area size of the virtual surface (plane), the storage volume and hence the contribution to the storage volume generated by the second portion is determinable (i.e. calculable).

The contribution of at least 10% of the total storage volume is definable under given boundary conditions. In particular, the contribution of at least 10% of the total storage volume under the critical pressure in the storage volume is achievable in the case where the ambient pressure is approximately 20° Celsius and the predetermined pressure is approximately 1013 hPa.

In particular, the exterior membrane is formed in such a way that the contribution to the total storage volume by the second portion under the critical pressure in the storage volume may be at least approximately 10%, in particular approximately at least 15% and more particularly at least 25% under the above described boundary conditions.#

The exterior membrane may in particular be made of a fibre-free material, i.e. the exterior membrane is not made of a material with inductile fibres. Moreover, the exterior membrane is made of a foil-like material. Moreover, the material of the exterior membrane may be an elastomer and/or a thermoplastic elastomer.

In gas storages, the exterior membrane may cover an area surface between 150 m$^2$ (square meter) and 1300 m$^2$ (square meter). Exterior membranes according to the present invention comprise in the force-free condition a width or a diameter of the gas storage of approximately 15 m to approximately 45 m (meter) or more.

By the present invention, the exterior membrane is formed for being expandable (for providing a volume contribution to the storage volume of at least 10% before failing) and shrinkable depending on the pressure of the gaseous medium in the storage volume. If the gas pressure in the storage volume decreases, the exterior membrane shrinks and reduces its area size, so that the storage volume is reduced. Hence, the pressure in the storage volume is kept in between predetermined pressure ranges due to the reduced area size of the exterior membrane. Thereby, a sufficient tension of the exterior membrane is still provided that prevents the exterior membrane from leaving its homogeneous shape. In other words, due to the stretch and expanding properties of the exterior membrane, the area size of the exterior membrane is adaptable and amendable to the respective pressure of a gaseous medium in the storage volume. Hence, if a tension of the exterior membrane is provided, e.g. fluttering of the exterior membrane in the wind may be prevented.

By the covering with the exterior membrane which is partially covered with the support structure according to the present invention, also large gas storage diameters may be covered with a stable exterior membrane which has a high stability against wind and snow loads. Moreover, due to the second portions of the exterior membrane which are not covered by the support structure, the exterior membrane may be elongated with its second portions by the different gas pressures inside the storage volume, so that the gas storage may still be fed in and drained off with gas. Moreover, the inventive solution provides a cost saving solution.

Summarizing, the covering according to the present invention comprises an elastic and stretchable exterior membrane which is mountable to an edge of the gas storage. The first portion of the exterior membrane is covered by the support structure, so that an elongation of the first portion is limited. The second portion of the exterior membrane is not covered by the support structure, so that a further elongation and expansion of the second portion is allowed. In particular, by the second portion which is contact-free with the support structure, a further expansion of the exterior membrane is possible in comparison to an embodiment which is fully covered by the support structure and thus only comprises first portions.

The stretchable exterior membrane provides a buffer volume by forming with its second portion the contribution to the storage volume. If there is a need of storing more gaseous medium in the storage volume, the second portion may expand and may thus provide the required additional volume. Hence, a storage with a flexible and adjustable volume for the gaseous medium is achieved.

According to a further exemplary embodiment the second portion has a projected area onto the ground along a vertical direction which may have a size of more than 75m$^2$, for example between substantially 200 m$^2$ to 300 m$^2$.

According to a further exemplary embodiment the second portion of the exterior membrane and the support structure are further formed in such a way that c) if an operating pressure value, which is between the first pressure value and the critical pressure value, in the storage volume is reached, the first portion of the exterior membrane contacts the support structure in the force transmitting manner and the second portion of the exterior membrane is contact-free with the support structure, wherein, if the operating pressure value storage volume is reached, the second portion is expandable such that a contribution to the storage volume of at least approximately 5% in comparison to the volume of the storage volume with the first pressure value is generated.

The operating pressure value defines a pressure in the storage volume which is the nominal pressure at which the covering is designed for operation. The operating pressure value the covering may be defined e.g. in the specifications defined by a manufacturer of the covering.

According to a further exemplary embodiment, the material of the exterior membrane is a synthetic rubber, in particular ethylene propylene diene monomer (EPDM). EPDM is a type of synthetic rubber and an elastomer. An EPDM elastomer is usable in a temperature range of approximately -40°C to approximately 110°C without failing.

According to a further exemplary embodiment, the exterior membrane is formed homogeneous. In particular, the exterior membrane may be formed of one piece or a plurality of sub-pieces, wherein in the force-free unexpanded condition, the exterior membrane may extend along a plane. In that case, the dome-like shape of the exterior membrane is formed by stretching of the exterior membrane if the pressure in the storage volume increases. By the stretchable membrane according to the present invention, in the original and unstretched state, the stretchable membrane may be plane, i.e. with a flat and substantial two dimensional shape. The curvature in an operating state of the gas storage is achieved by stretching the stretchable membrane due to a respective gas pressure in the storage volume. Hence, tailoring of the exterior membrane is easy.

According to a further exemplary embodiment, the exterior membrane is formed in such a way that that the exterior membrane has in the second portion a stretch ratio between the first pressure value in the storage volume and the operating pressure value in the storage volume of more than 1,1, e.g. for achieving the contribution of at least 10 % to the storage volume (i.e. measured under a temperature of 23° degree Celsius).

The expanded condition of the second portion of the exterior membrane defines the length, the diameter or preferably the area size of the second portion of the exterior membrane in which an operating pressure in the storage volume is applied.

The stretch ratio (extension ratio) is a measure of the extensional or normal elongation of a differential line element or of a differential area of an elemental area. The stretch ratio may be defined as described above and by the following formula:

$$\lambda = \frac{a}{A}$$

wherein

λ　is the stretch ratio;
a　is the area of the second portion of the exterior membrane under the operating pressure value in the storage volume; and
A　is the area of the second portion of the exterior membrane under the first pressure value in the storage volume.

The area size of the second portion of the exterior membrane may be illustrated and calculated by a surface integral that is taken over the surface of the second portion of the exterior membrane. Hence, curved surfaces of the second portion of the exterior membrane that describes e.g. a shape of half hollow ball may be defined and calculated as well, so that the stretch ratio may be defined. Alternatively, the stretch ratio may as well be expressed by a ratio between length differences of the second portion of the exterior membrane along its surface between a condition under the first pressure value in the storage volume and the condition under the operating pressure value in the storage volume. The length of the second portion of the exterior membrane may be defined by a length of a line between two opposing points which are located on an edge portion of the second portion, wherein the line runs through the center point of the second portion. Hence, the length of the line between the two opposing points, which line runs along the surface through the center point of the second portion of the exterior membrane, may be calculated by a line integral. For example, when taking a line between the two opposing points, the connection line along the surface of the exterior membrane may form for example a curved shape, such as a parable or a half circle, for example.

In particular, the stretch ratio according to the present invention is approximately more than 1,1. In particular, the stretch ratio of the exterior membrane is between approximately 1,1 and approximately 4, and more particularly between approximately 2 and approximately 3. The values for the stretch value be in particular be valid at ambient temperatures of approximately -40°C to approximately 100°C, and more particularly between approximately -20°C to approximately 70°C.

Additionally, in a further exemplary embodiment, the exterior membrane may comprise furthermore, for achieving the contribution of at least 10 % to the storage volume, a material with an ultimate elongation of more than 100% (i.e. measured under a temperature of 23° degree Celsius).The ultimate elongation strength is a material property and is defined by:

$$A = \frac{\Delta L}{L_0} \bullet 100\%$$

wherein

A        is the ultimate elongation,
$\Delta L$        is the length difference between the start length (area) of the exterior membrane under a force-free unexpanded condition of the exterior membrane and the length (area) of the exterior membrane under the critical pressure value in the storage volume, at which the exterior membrane fails;
$L_0$        is the start length (area) of the exterior membrane under a force-free unexpanded condition.

The force-free unexpanded condition of the exterior membrane defines the condition of the exterior membrane, at which i.e. no tractive forces or pressure forces caused due to a pressure difference between the pressure of the storage volume and an ambient pressure act. Under the term force-free unexpanded condition a condition is described, where generally only e.g. the weight force of the respective membrane loads the respective membrane.

The length is measurable between two opposing points which are located at opposing edges of the exterior membrane. Alternatively, the area size of the exterior membrane is measurable under the force-free unexpanded condition and under the critical pressure value in the storage volume.

An ultimate elongation of more than 100% defines a material, which is adapted to withstand an elongation of its length of more than the double length (A=100%) of the start length.

According to a further exemplary embodiment, the support structure comprises elongated restraining elements, particularly cables, belts and/or ropes.

In the following embodiments, the run of elongated restraining elements is described in various exemplary embodiments. In particular, the run of the elongated restraining elements, such as restraining cables, may form a certain portion of the support structure as described below.

In a first exemplary embodiment, at least two of the elongated restraining elements run parallel along the exterior membrane. In particular, a projected area of the exterior membrane onto the ground along a vertical direction is projectable, onto which projected area the projected contours of the elongated restraining elements run along the projected area in a parallel manner. In particular, if the exterior membrane forms a hollow half ball shape, the elongated restraining elements may run along a great circuit from an edge of the support structure to a centre of the exterior membrane. If the exterior membrane is e.g. a half ball like shape, the elongated restraining elements may cross each other in a top section of the exterior membrane.

According to a further exemplary embodiment, first elongated restraining elements of the elongated restraining elements and second elongated restraining elements of the elongated restraining elements are arranged in such a way that the first elongated restraining elements cross the second elongated restraining elements, so that between the first elongated restraining elements and the second elongated restraining elements sub portions of the second portion are formed. By the term "crossing" it is described, that the first elongated restraining elements and the second elongated restraining elements cross each other. Further contact by the term "crossing" it is also described that two end sections of the first elongated restraining elements and the second elongated restraining elements ends in one common point, for example. In particular, a projected area of the exterior membrane onto the ground along a vertical direction is projectable, onto which projected area the projected contours of the elongated restraining elements run along the projected area in a non-parallel manner. In each sub portion, an exceeding of the predetermined pressure value in the storage volume leads to a stretching of the sub portions of the second portion, so that a volume gain is generatable. In particular, the additional volume respectively the contribution of the second portion to the total storage volume of approximately 10% may be achieved by a sum of sub contributions of the sub portion to the storage volume.

According to a further exemplary embodiment, at least one of the sub portions of the second portion has an area size of approximately more than $75 m^2$, if the first pressure value in the storage volume is reached. The first elongated restraining elements and the second elongated restraining elements may also be arranged in such a way that respective area sizes of the at least one sub portion of the second portion or the sum of the sub portions of the second portion is larger than $100\ m^2$ (square meter), if a first pressure value in the storage volume is reached. In particular, at least one sub portion of the second portion or the sum of the sub portions of the second portion is approximately $200\ m^2$ to $500\ m^2$ if the first pressure value in the storage volume is reached.

According to a further exemplary embodiment, the first elongated restraining elements cross the second elongated restraining elements in such a way that the sub portions of the second portions surrounded by the respective first elongated restraining elements, the second elongated and/or an upper edge of a sidewall of the gas storage restraining elements have a surface form of a polygon with at least three corners. Hence, each sub-potion may have a shape of a triangle, a rectangle, a pentagon or a hexagon. Alternatively, the sub portion my also comprise a shape of a circle or an ellipse. The surface of the sub portions of the second portions may be also surrounded by an edge of the gas storage, the respective first elongated restraining elements and respective second elongated restraining elements. In particular, a projected area of the exterior membrane onto the ground along a vertical direction is projectable, onto which projected area the projected contours of the first and second elongated restraining elements run along the projected area and thereby forming the above defined surface forms.

According to a further exemplary embodiment, the elongated restraining elements form a mesh to which the first portion of the exterior membrane is alignable.

In particular, according to a further exemplary embodiment, the mesh has mesh openings, wherein each mesh opening is formed with a size that is smaller than the size of each of the respective sub portions of the second portion.

In particular, the mesh openings are small enough, such that the mesh of the support structure absorbs forces from the first portion which is pressed against the mesh, if the pressure in the storage volume exceeds the first pressure value.

In particular, the size of each mesh opening may be smaller than 100 m$^2$, in particular smaller than 10 m$^2$.

In particular, the mesh may be formed by a plurality of crossing first and second elongated restraining elements, wherein the sub portions of the first portions surrounded by the first and second elongated restraining elements have a surface form of a triangle, a rectangle, a pentagon, a hexagon, a circle, an ellipse or a polygon. In particular, a projected area of the exterior membrane onto the ground along a vertical direction is projectable, onto which projected area the projected contours of the first and second elongated restraining elements run along the projected area and thereby forming the above defined shape of the mesh openings of the mesh.

According to a further exemplary embodiment, the first portion is an edge portion extending from an edge of the exterior membrane in the direction to a centre of the exterior membrane. The second portion is a centre portion of the exterior membrane extending from the first portion to the centre of the exterior membrane. Hence, the support structure is mounted to the storage, in particular to an upper edge of a sidewall of the storage and extends to the centre of the storage respectively to a centre of the exterior membrane. Hence, the edge portion runs around the edge of the sidewall of the storage at which a more comfortable and robust fixation of the support structure is achievable. The second portion extends around a centre section of the exterior membrane and comprises the center section. Further support structures for the second portions are not necessary, so that the second portion may be spaced from an edge of the storage. Hence, by the arrangement of the above-described edge portion and centre portions, a large area of the exterior membrane may be applied.

According to a further exemplary embodiment, the storage further comprises a ground plate and a sidewall mounted to the ground plate. The exterior membrane and/or the support structure is/are mounted to the (upper edge of the) sidewall so that the storage volume is enveloped by virtual surface (plane) onto which the upper edges of the sidewall are located and the exterior membrane.

In a further exemplary embodiment, the exterior membrane is mounted to an edge of a hole in a ground (e.g. a syncline), so that the storage volume is formed by the exterior membrane and a surface of the hole in the ground.

According to a further exemplary embodiment, the storage further comprises a level indicator for measuring the gas compartment in the storage volume. The level indicator may measure the gas compartment for example by measuring the position of the exterior membrane by an ultrasonic measurement. By using an ultrasonic level indicator, the ultrasonic level indicator may be mounted to the top of the exterior membrane, so that the distance to the ground or to the biomass in the storage may be measured.

According to a further aspect of the present invention, the storage for storing a gaseous medium is presented. The storage comprises a membrane for at least partially enveloping a storage volume and a gas supply unit which is connected to the storage volume for supplying supporting gas to the storage volume, so that the gas pressure in the storage volume is prevented from falling below a predetermined nominal gas pressure.

The supporting gas may be selected from one of the group consisting of inert gases, natural gases, helium gases, halogen gases and air. Moreover, the support gas may be an industrial gas which is of the same condition as the industrial gas regularly stored in the storage.

If the gas pressure in the storage volume falls below a predetermined nominal gas pressure, the support gas is fed to the storage volume, so that the gas pressure in the storage volume increases above the nominal gas pressure. Hence, the supply unit prevents the membrane from folding and losing its tension. The gaseous medium in the storage volume will be mixed with the supporting gas supplied by the gas supply unit.

According to a further exemplary embodiment, the storage comprising the membrane and the gas supply unit is a storage as described above.

It has to be noted that embodiments of the invention have been described with reference to different subject matters. In particular, some embodiments have been described with reference to apparatus type claims whereas other embodiments have been described with reference to method type claims. However, a person skilled in the art will gather from the above and the following description that, unless other notified, in addition to any combination of features belonging to one type of subject matter also any combination between features relating to different subject matters, in particular between features of the apparatus type claims and features of the method type claims is considered as to be disclosed with this application.

Brief Description of the Drawings

The aspects defined above and further aspects of the present invention are apparent from the examples of embodiment to be described hereinafter and are explained with reference to the examples of embodiment. The invention will be described in more detail hereinafter with reference to examples of embodiment but to which the invention is not limited.

Fig. 1 shows a view of a storage for a gaseous medium according to an exemplary embodiment of the present invention;

Fig. 2 shows a perspective view of a gas storage with sidewalls, to which upper edge a covering is mounted according to an exemplary embodiment of the invention;

Fig. 3 shows a perspective view of a storage with sidewalls, to which upper edge a covering with a mesh shaped support structure is mounted according to an exemplary embodiment of the invention;

Fig.4 shows a storage with rectangular-shaped second sub portions according to an exemplary embodiment of the invention;

Fig. 5 shows a top view of a storage, wherein the central second sub portion has generally a hexagonal shape and the second sub portions surrounding the central second sub portion have generally a pentagonal shape, according to an exemplary embodiment of the invention;

Fig. 6 to Fig. 9 show top views of storages, wherein the support structures form a mesh according to exemplary embodiments of the present invention;

Fig. 10 shows a conventional gas storage with an exterior membrane;

Fig. 11 shows a conventional gas storage with an exterior membrane and a conventional support structure; and

Fig. 12 shows a conventional gas storage with an exterior membrane showing three different positions of the exterior membrane.

Detailed Description

The illustrations in the drawings are schematically. It is noted that in different figures, similar or identical elements are provided with the same reference signs.

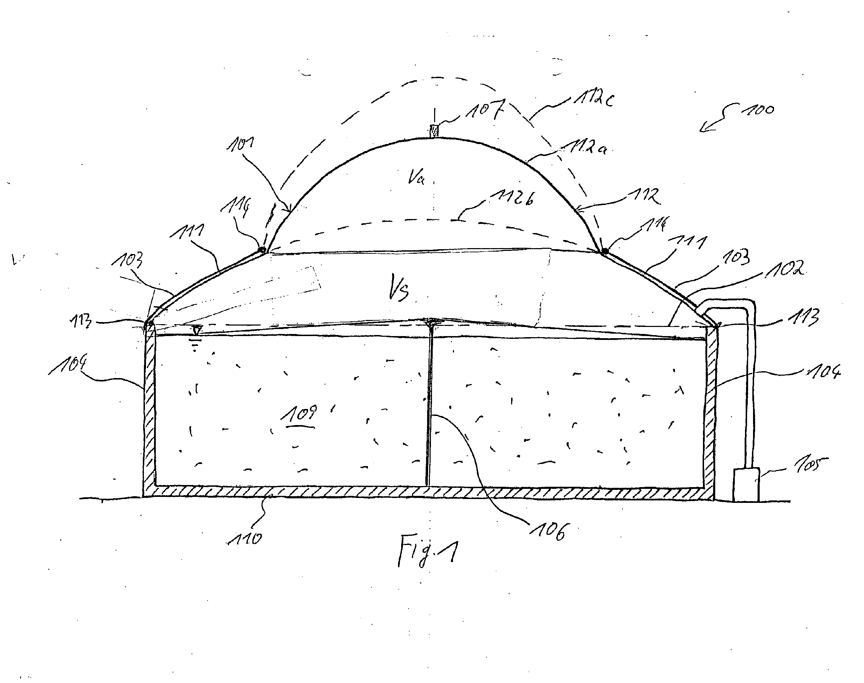

**Fig. 1** shows a storage 100 for a gaseous medium to which a covering is mounted. The covering comprises an exterior membrane 101 and a support structure comprising elongated restraining elements 103. The exterior membrane 101 at least partially envelops a storage volume Vs of the storage 100. The exterior membrane 101 is made of an elastomeric material, so that a shape and an area size of the exterior membrane 101 are adaptable to a pressure of the gaseous medium in the storage volume Vs.

The storage volume Vs denotes the gas compartment which is generated between the exterior membrane 101 and a virtual surface (plane) 102, within virtual surface (plane) 102 an surrounding upper storage edge 113 of the gas storage is located. To the upper storage edge 113 the exterior membrane 101 is mounted. The storage volume Vs describes the volume enveloped by the exterior membrane 101 and the virtual surface 102 at the upper edge 113 of the gas storage, in particular the upper edge 113 of sidewalls 104 of the gas storage 100.

The support structure is mounted over the exterior membrane 101 in such a way that a) if a first pressure value in the storage volume Vs is reached, a first portion 111 of the exterior membrane 101 contacts the support structure in a force transmitting manner and a second portion 112b of the exterior membrane 101 is contact-free with the support structure, and b) if a critical pressure value storage volume Vs is reached, at least one of the support structure and the exterior membrane 101 fails, wherein if the critical pressure value in storage volume Vs is reached, the second portion 112c is expanded such that a contribution Va to the storage volume Vs of at least 10% in comparison to the volume of the storage volume Vs with the first pressure value is generated.

The support structure may be formed of elongated restraining elements 103, in particular of cables, ropes or belts. As shown in Fig. 1, the elongated restraining elements 103 run from the upper edge 113 at the sidewall 104 of the storage 100 to a centre portion of the storage 100. The first portion 111 of the exterior membrane 101 is aligned at the support structure, in particular at the elongated restraining elements 103, in a force transmitting manner if the first pressure value in the storage volume Vs is exceeded so that a force may be transmitted to the elongated restraining elements 103. In the centre section, the exterior membrane 101 comprises the second portion 112 that is not in contact with the elongated restraining elements 103 and is thus not restricted in its flexibility and expandability by the support structure.

As shown in Fig. 1, the second portion 112 is shown in a condition 112a under the operating pressure value, in a condition 112b under the first pressure value and in a condition 112c under the critical pressure value.

The condition 112a of the second portion 112 under the operating pressure value is shown with the solid line in Fig. 1. The second portion 112 is stretched because the gas pressure in the storage volume Vs exceeds the first pressure value. Hence, the second portion 112 is stretched and forms a cupola or dome-like shape, and if the first pressure value is exceeded, the stretched and expanded condition 112a of the second portion 112 generates a contribution Va to the storage volume Vs. The second portion 112 is stretchable under the critical pressure value in such a way that the contribution Va to the storage volume Vs generated by the second portion 112c is at least approximately 10% of the total storage volume Vs before the membrane fails.

In contrary, as can be taken from Fig. 1, the first portion 111 is aligned at the elongated restraining elements 103 and does not expand for generating an additional volume contribution but contacts the elongated restraining elements 103 in a force transmitting manner if the first pressure value in the storage volume Vs is exceeded.

Fig. 1 shows that the exterior membrane 101 is expandable, so that due to a gas pressure in the storage volume Vs, the exterior membrane 101 and in particular the second portion 112 is adapted with its shape and area size to the pressure of the gaseous medium and the storage volume Vs.

The stretch ratio of the second portion 112 may be defined as described above, wherein the stretch ratio is a measure of the extensional or normal elongation of a differential line element or of a differential area of an elemental area. The stretch ratio may be defined as described above and by the following formula:

$$\lambda = \frac{a}{A}$$

wherein

λ    is the stretch ratio;
a    is the area of the second portion of the exterior membrane under the operating pressure value in the storage volume; and
A    is the area of the second portion of the exterior membrane under the first pressure value in the storage volume.

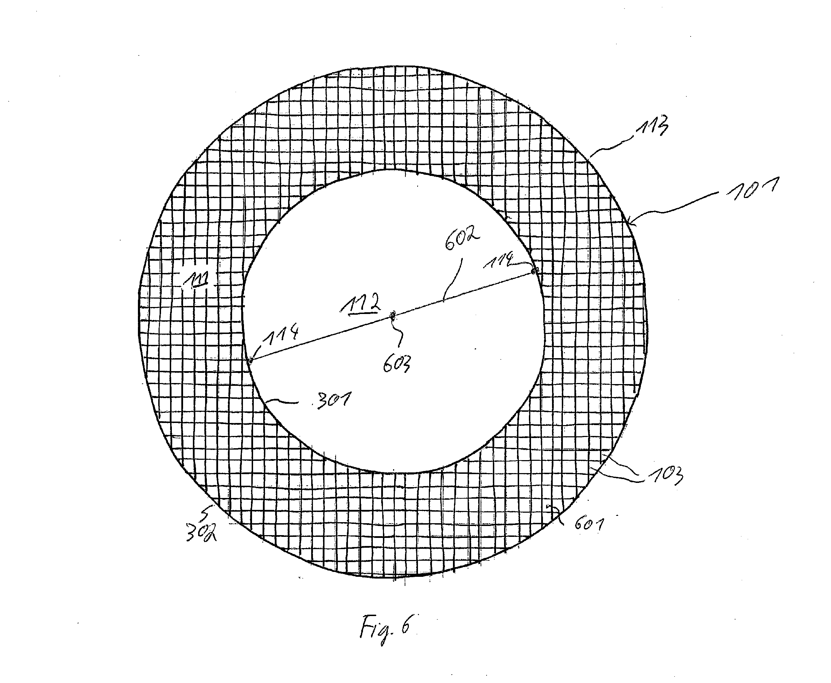

The area size of the second portion 112 of the exterior membrane may be illustrated and calculated by a surface integral that is taken over the surface of the second portion 112 of the exterior membrane 101. Hence, curved surfaces of the second portion 112 of the exterior membrane 102 that describes e.g. a shape of half hollow ball may be defined and calculated as well, so that the stretch ratio may be defined. Alternatively, the stretch ratio may as well be expressed by a ratio between length differences of the second portion 112 of the exterior membrane 102 along its surface between a condition 112b under the first pressure value in the storage volume Vs and the condition 112a under the operating pressure value in the storage volume. The length of the second portion 112 of the exterior membrane may be defined by a length of a line 602 (see Fig. 6) between two opposing points 114 which are located on an edge portion of the second portion 112, wherein the line 602 runs through the center point 603 (see Fig. 6) of the second portion 112.

Fig. 1 shows furthermore the storage 100 with the sidewall 104 and the ground plate 110. The storage volume Vs is defined between the exterior membrane 101 and the virtual surface (plane) 102 which connects the upper storage edge 113 of the gas storage, to which upper storage edge 113 the exterior membrane 101 is mounted. The industrial gas may comprise for example a biogas, a natural gas or liquid gas.

Optionally, in order to prevent a contact of the exterior membrane 101 with the ground plate 110 or the biomass 109, an inner support structure 106 may be installed into the storage 100. The inner support structure 106 may comprise one or more supporting masts that support the exterior membrane 101. Moreover, the inner support structure 106 may comprise a plurality of supporting belts or cables onto which the exterior membrane 101 may be aligned if the gas pressure in the storage volume Vs falls below the nominal gas pressure value, i.e. the first pressure value.

A support gas supply unit 105 may optionally be installed and connected to the storage 100. The support gas supply unit 105 supplies support gas into the storage volume Vs. Hence, a pressure in the storage volume Vs may be kept generally in between predetermined pressure ranges even when the storage volume Vs increases. Hence, the tension and the size of the exterior membrane 101 may be sustained by keeping the pressure in the storage volume Vs generally in between a predetermined pressure range. The support gas may be for instance an inert gas, for example.

Further referring to Fig.1, a level indicator 107 may be mounted to, e.g. a top, of the exterior membrane 101 or to any other location onto the exterior membrane. The top of the exterior membrane 101 may be in general the highest point of the storage 100. The level indicator 107 may measure the storage volume Vs for example by measuring the position of the exterior membrane 101 by an ultrasonic measurement or by measuring the pressure in the storage volume Vs.

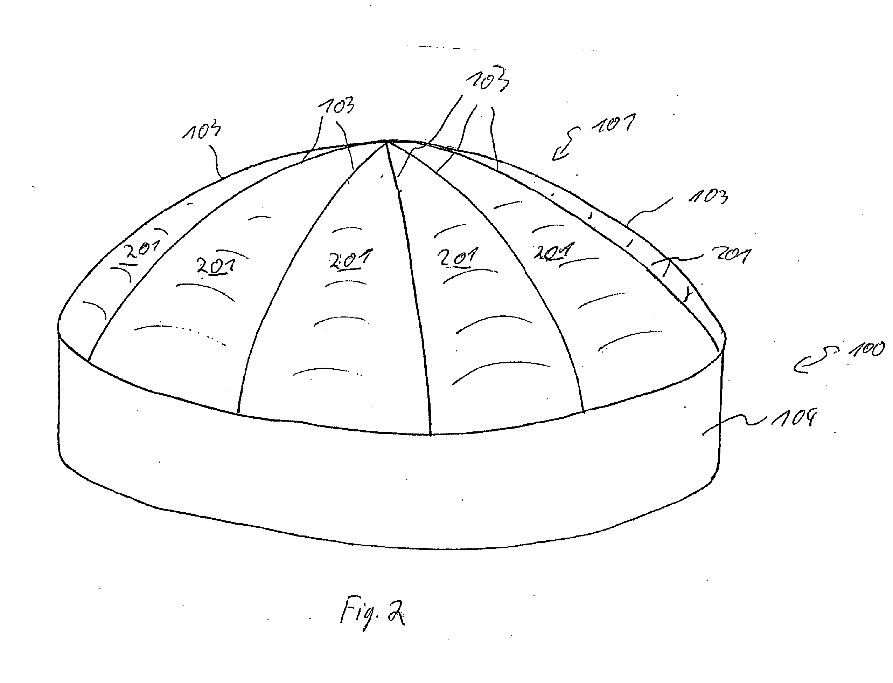

Fig. 2 shows a storage 100 with the exterior membrane 101, wherein elongated restraining elements 103 of the support structure are mounted to upper edges of sidewalls 104 of the storage 100. The elongated restraining elements 103 are for example flexible ropes, belts or cables. The elongated restraining elements 103 are mounted over the exterior membrane 101, so that the exterior membrane 101 is in a force transmitting contact with its first portions 111 (not shown in Fig. 2) at the elongated restraining elements 103 of the support structure when the exterior membrane 101 expands due to the gas pressure in the storage volume Vs exceeding the first pressure value.

Hence, the exterior membrane 101 may be reinforced by the elongated restraining elements 103 of the outer support structure. The elongated restraining elements 103 may form various patterns and may comprise different extension directions.

Moreover, between the elongated restraining elements 103 sub portions 201 of the second portion 112 are formed. The distance and the area between the elongated restraining elements 103 are large enough so that the sub portions 201 of the exterior membrane 101 are stretched and expanded by a pressure that is equal or exceeds the first pressure value of the storage volume Vs. The sum of the contribution Va of each sub portion 201 forms the total contribution Va of more than 10% to the storage volume Vs, if the pressure in the storage volume Vs reaches the critical pressure value. The contribution Va may be larger than at least approximately 10% of the total storage volume Vs.

The first portions are defined by the area of the exterior membrane 101 that is in contact with the elongated restraining elements 103, in particular the first portions are the areas of the exterior membrane 101 that is located below the elongated restraining elements 103 and thus not shown in some figures.

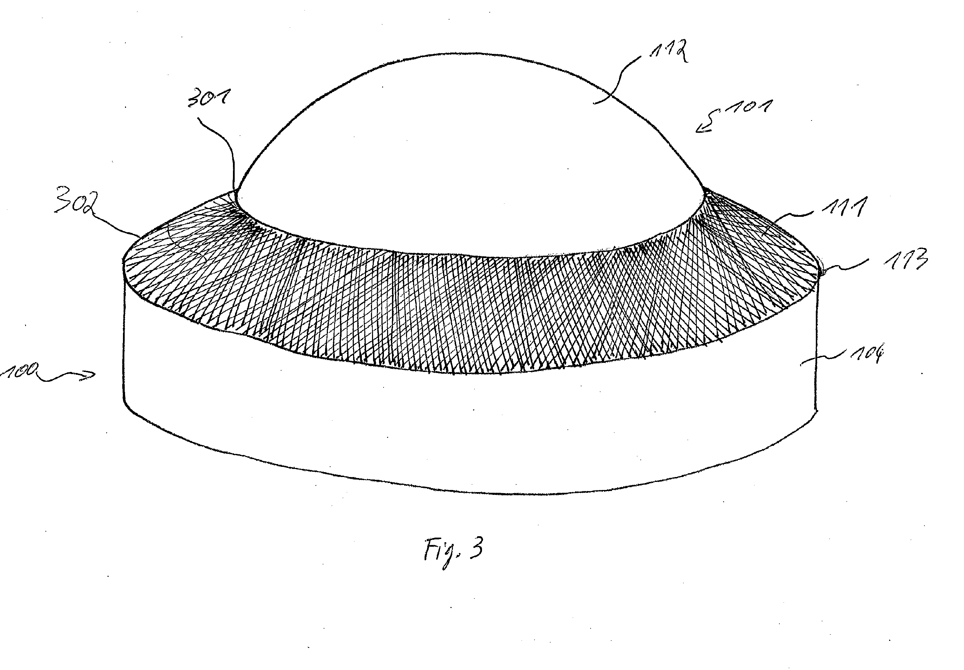

**Fig. 3** shows a perspective view of an exemplary embodiment of the storage 100 with sidewalls 104, to which upper edge 113 the covering is mounted, wherein the second portion 112 of the exterior membrane 101 forms a hollow half ball shape. The support structure is formed by a plurality of elongated restraining elements 103 that forms a mesh located over the first portion 111 of the exterior membrane 101. For example, the elongated restraining elements 103 extend between an inner circular elongated restraining element 301 and an outer circular restraining element 302.

As shown in Fig. 3, the first portion 111 which is alignable in a force transmitting manner and in contact with the mesh formed by the elongated restraining elements 103 extends from the upper edge 113 to the inner circular restraining element 301 of the support structure. The second portion 112 comprises a centre portion of the exterior membrane 101 and extends between the first portion 111 (i.e. the inner circular elongated restraining element 301) around the centre 603 (see Fig. 6). The elongated restraining elements 103 run between the inner elongated restraining element 301 and the outer circular restraining element 302. Such a mesh comprising the inner elongated restraining element 301 and the outer circular restraining element 302 between which elongated restraining elements 103 run is easy to manufacture so that the manufacturing costs are reduced. Moreover, the above described mesh design provides a homogeneous and uniformly load transmission from the first portion 112 to the gas storage 100.

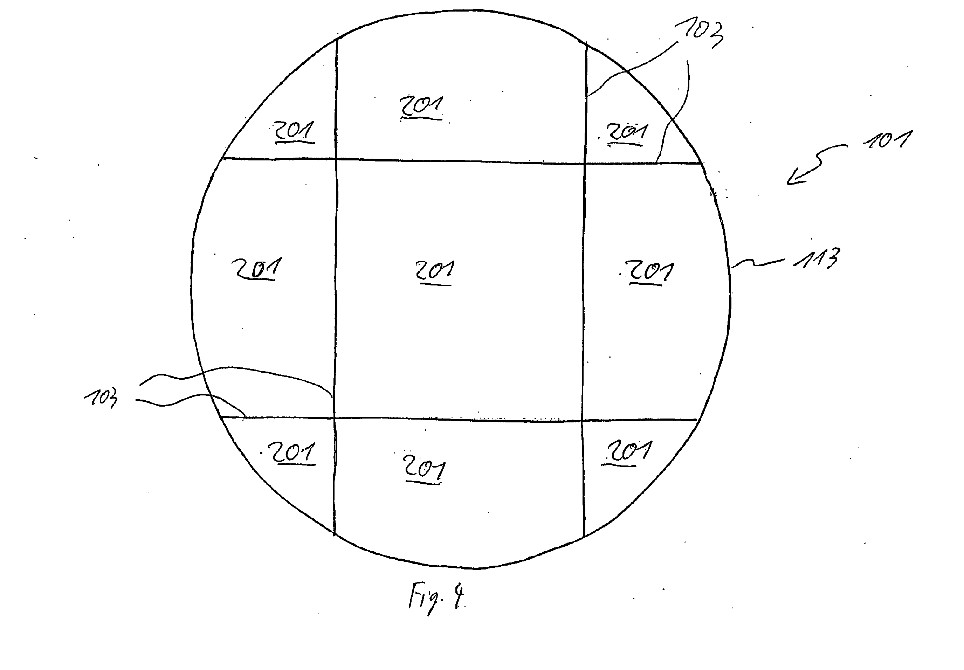

**Fig. 4** illustrates a projected area of the exterior membrane 101 and the support structure, which projected area is formed by a projection of the exterior membrane 102 and the support structure onto the ground along a vertical direction. The elongated restraining elements 103 define large sub portions 201. In particular, the projected contours of the first and second elongated restraining elements 103 run along the projected area and thereby forming the rectangular shape.

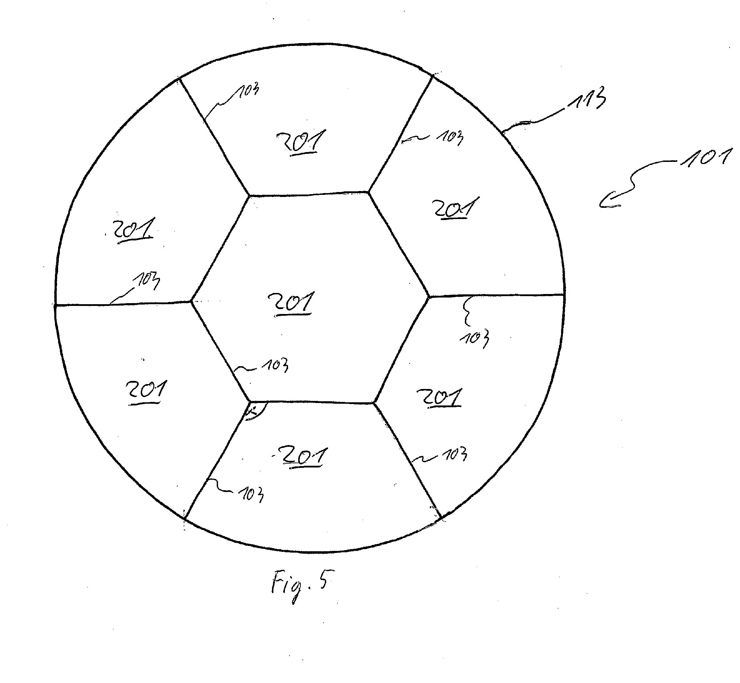

**Fig. 5** shows a further exemplary embodiment of the exterior membrane 101. Fig. 5 shows a projected area of the exterior membrane 101 and the support structure, which projected area is formed by a projection of the exterior membrane 102 and the support structure onto the ground along a vertical direction. The elongated restraining elements 103 form a hexagonal shaped central sub portion 201 of the second portion 112. Furthermore, the elongated restraining elements 103 form second sub portions 201 which surrounds the central second sub portion 201, wherein the surrounding second sub portion 201 have generally a pentagonal shape. In particular, onto the projected area the projected contours of the first and second elongated restraining elements 103 run along and thereby defining an angle $\alpha$ between each other. The angle $\alpha$ of the crossing or touching elongated restraining elements 103 may be between approximately 45° and 120° degree.

**Fig. 6** shows an exemplary embodiment of the storage 100 as shown in Fig.3, wherein the support structure is formed by a plurality of elongated restraining elements 103 that forms a mesh located over the first portion 111 of the exterior membrane 101. For example, the elongated restraining elements 103 extend between the inner circular elongated restraining element 301 and the outer circular restraining element 302. Fig. 6 shows a projection of the exterior membrane 102 and the support structure onto the ground along a vertical direction, onto which projected area the projected contours of the first and second elongated restraining elements 103 run along. The length of the second portion 112 of the exterior membrane may be defined by the length of the line 602 between two opposing points 114 which are located on an edge portion of the second portion 112, wherein the line 602 runs through the center point 603 (see Fig. 6) of the second portion 112.

In the exemplary embodiment of Fig. 6, the elongated restraining elements 103 form small rectangle mesh openings 601, wherein the mesh openings 601 are that small, that the exterior membrane 101 in the first portion 111 does not (or at least marginally) stretch and expand through the mesh openings 601, when the pressure of the storage volume Vs is equal or exceeds the first pressure value.

As shown in Fig. 6, the first portion 111 which is alignable to the mesh formed by the elongated restraining elements 103 extends from an edge portion 113 to a centre 603 of the exterior membrane 101. The second portion 112 forms a centre portion of the exterior membrane 101 and ranges between the first portion 111 and the centre 603. The elongated restraining elements 103 run between the inner elongated restraining element 301 and the outer circular restraining element 302 and form the mesh. The mesh design provides a homogeneous and uniformly load transmission from the first portion 111 to the gas storage 100.

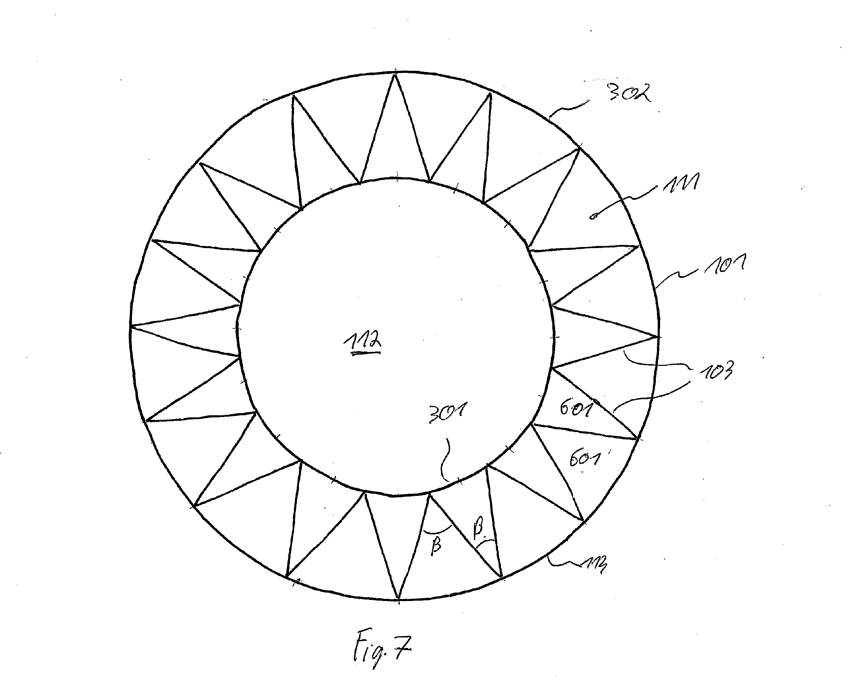

**Fig. 7** shows a further pattern of a mesh formed by the elongated restraining elements 103. For example, the elongated restraining elements 103 extend between the inner circular elongated restraining element 301 and the upper edge 113 of sidewall 104 or the outer circular restraining element 302. Between the inner and the outer circular elongated restraining elements 301, 302, further elongated restraining elements 103 run in circumferential direction along a zigzag path and forms triangular shaped mesh openings 601. In particular, a projected area of the exterior membrane 101 onto the ground along a vertical direction is projectable, onto which projected area the projected contours of the elongated restraining elements 103 run along the projected area and thereby forming the triangular shaped mesh openings 601 and defining an angle β between each other. In particular, an angle β between two crossing elongated restraining elements 103, wherein each of the two crossing elongated restraining elements 103 ends in one common point, is approximately 10° to 40°. In particular, the angle β is the smaller angle of the angles between two crossing elongated restraining elements 103.

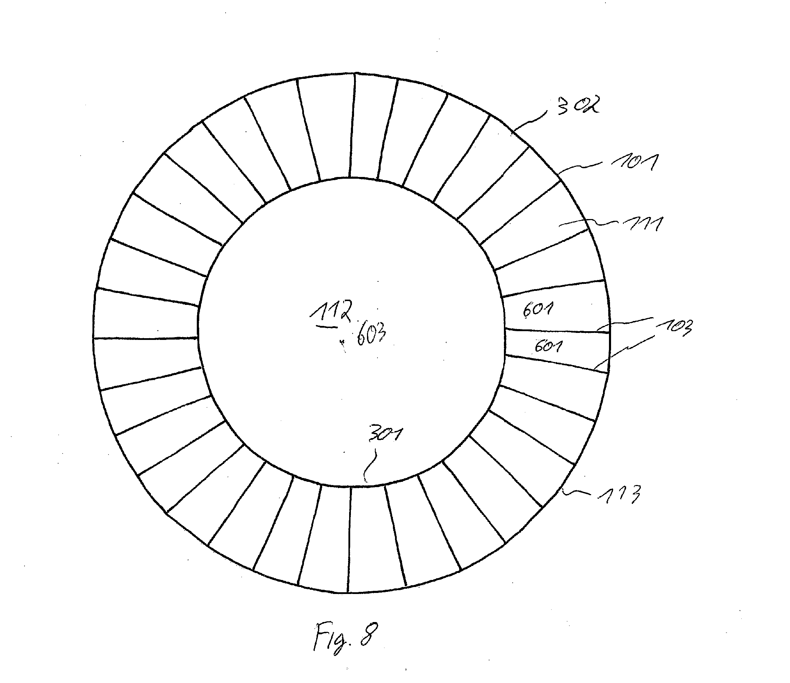

As shown in **Fig. 8,** a projected area of the exterior membrane 101 and the support structure, which projected area is formed by a projection of the exterior membrane 102 and the support structure onto the ground along a vertical direction is shown, wherein the elongated restraining elements 103 extend between the inner circular elongated restraining element 301 and the upper edge 113 of sidewall 104 or the outer circular restraining element 302, wherein the elongated restraining elements 103 do not cross and contact each other.

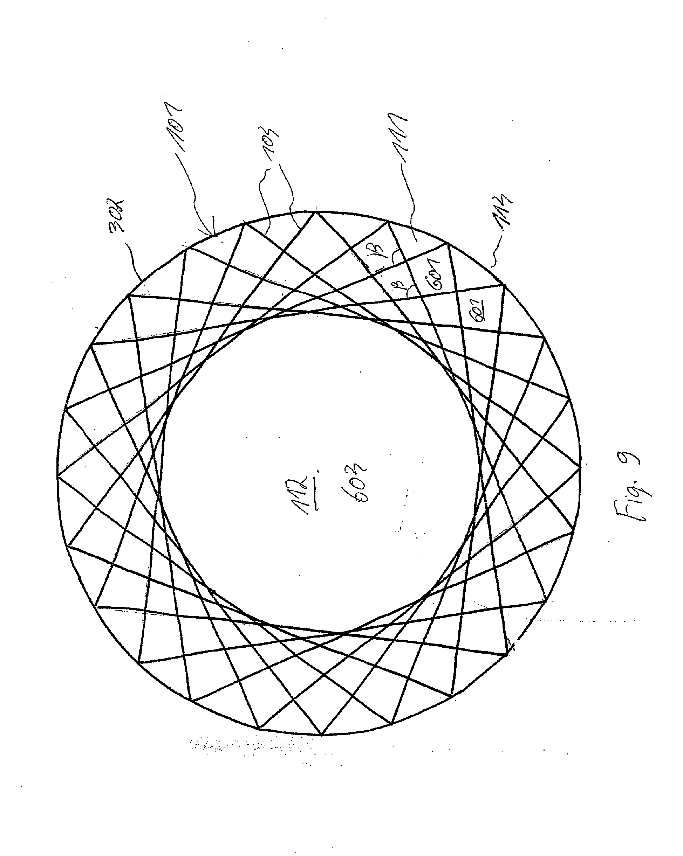

**Fig. 9** illustrates a projected area of the exterior membrane 101 and the support structure, which projected area is formed by a projection of the exterior membrane 102 and the support structure onto the ground along a vertical direction. As shown in Fig. 9, the mesh is formed by the plurality of elongated restraining elements 103 that run along the projected area straight from one point on the upper edge 113 of sidewall 104 to a further point on the upper edge 113. The elongated restraining elements 103 run straight on the projected area between the edges 113 in such a way that the elongated restraining elements 103 thereby passing and forming the centre section 603 of the exterior membrane 101. The angle β onto the projected area between the two crossing elongated restraining elements 103 may be approximately 45° to 120°, for example.

It should be noted that the term "comprising" does not exclude other elements or steps and "a" or "an" does not exclude a plurality. Also elements described in association with different embodiments may be combined. It should also be noted that reference signs in the claims should not be construed as limiting the scope of the claims.

List of reference signs:

| | |
|---|---|
| 100 | storage |
| 101 | exterior membrane |
| 102 | surface plane |
| 103 | elongated restraining element |
| 104 | sidewall |
| 105 | support gas supply unit |
| 106 | inner support structure |
| 107 | level indicator |
| 109 | biomass |
| 110 | ground plate |
| 111 | first portion |
| 112 | second portion |
| 112a | condition of second portion under the operating pressure value |
| 112b | condition of second portion under the first pressure value |
| 112c | condition of second portion under the critical pressure value |
| 113 | upper edge of sidewall |
| 201 | sub portion of the second portion |
| 301 | inner circular elongated restraining element |
| 302 | outer circular elongated restraining element |
| 601 | mesh opening |
| 602 | line through centre of exterior membrane |

603    centre of exterior membrane

1001    conventional exterior membrane

1002    storage volume

1003    conventional inner support structure

1004    conventional outer support structure

Vs    storage volume

Va    contribution to the storage volume generated by second portion

α    angle between two crossing elongated restraining elements in the second portion

β    angle between two crossing elongated restraining elements in the first portion

**Claims**

1. Covering for a gas storage, the covering comprising an exterior membrane (101) for at least partially enveloping a storage volume (Vs) of the storage (100), wherein the exterior membrane (101) is made of an elastomeric material, so that a shape and an area size of the exterior membrane (101) is adaptable to a pressure of the gaseous medium in the storage volume (Vs), and
a support structure mounted over the exterior membrane (101), wherein the exterior membrane (101) and the support structure are formed in such a way that,

   a) if a first pressure value in the storage volume (Vs) is reached, a first portion (111) of the exterior membrane (101) contacts the support structure in a force transmitting manner and a second portion (112) of the exterior membrane (101) is contact-free with the support structure, and
   b) if a critical pressure value in the storage volume (Vs) is reached, at least one of the support structure and the exterior membrane (101) fails, wherein if the critical pressure value in storage volume (Vs) is reached, the second portion (112) is expandable such that a contribution (Va) to the storage volume (Vs) of at least 10% in comparison to the volume of the storage volume (Vs) with the first pressure value is generated.

2. Covering according to claim 1,
wherein the exterior membrane (101) and the support structure are further formed in such a way that,

   c) if an operating pressure value, which is between the first pressure and the critical pressure, in the storage volume (Vs) is reached, the first portion (111) of the exterior membrane (101) contacts the support structure in the force transmitting manner and the second portion (112) of the exterior membrane (101) is contact-free with the support structure,

wherein, if the operating pressure value storage volume (Vs) is reached, the second portion (112) is expandable such that a contribution (Va) to the storage volume (Vs) of at least 5% in comparison to the volume of the storage volume (Vs) with the first pressure value is generated.

3. Covering according to claim 1 or 2,
wherein the material of the exterior membrane (101) is a synthetic rubber, in particular ethylene propylene diene monomer.

4. Covering according to one of the claims 1 to 3,
wherein the exterior membrane (101) is formed in such a way that the exterior membrane (101) has in the second portion a stretch ratio between the first pressure value in the storage volume (Vs) and the operating pressure value in the storage volume (Vs) of more than 1,1.

5. Covering according to one of the claims 1 to 4,
wherein the support structure comprises elongated restraining elements (103), particularly cables, belts and/or ropes.

6. Covering according to claim 5,
wherein first elongated restraining elements of the elongated restraining elements (103) and second elongated restraining elements of the elongated restraining elements (103) are arranged in such a way that the first elongated restraining elements cross the second elongated restraining elements, so that between the first elongated restraining elements and the second elongated restraining elements sub portions (201) of the second portion (112) are formed.

7. Covering according to claim 6,
wherein at least one of the sub portions (201) of the second portion (112) has an area size of more than 75m$^2$, if the first pressure value in the storage volume (Vs) is reached.

8. Covering according to claim 6 or 7,
wherein the first elongated restraining elements cross the second elongated restraining elements in such a way that the sub portions (201) of the second portion surrounded by the respective first elongated restraining elements, the second elongated restraining elements and/or an upper edge of a sidewall (104) of the gas storage (100) have a surface form of a polygon with at least three corners.

9. Covering according to claim 8,
wherein the elongated restraining elements (103) form a mesh to which the first portion (111) of the exterior membrane (101) is alignable.

10. Covering according to claim 9,
wherein the mesh has mesh openings (601),
wherein each mesh opening (601) is formed with a size that is smaller than the size of each of the respective sub portions of the second portion.

11. Covering according to one of the claims 1 to 10,
wherein the first portion (111) is an edge portion extending from an edge (113) of the exterior membrane (101) in a direction to a centre (603) of the exterior membrane (101),
wherein the second portion (112) is a centre portion of the exterior membrane (101) extending from the first portion (111) to the centre (603) of the exterior membrane (101).

12. Gas storage (100) for storing a gaseous medium, the gas storage comprising
a covering as set forth in one of the claims 1 to 11, a ground plate (110), and
a side wall (104) mounted to the ground plate (110), wherein the exterior membrane (101) and/or the support structure is/are mounted to the side wall (104), so that the storage volume (Vs) is enveloped by the side wall (104), the exterior membrane (101) and the ground plate (110).

13. Method of manufacturing a covering for a gas storage, the method comprising
at least partially enveloping a storage volume (Vs) of the storage (100) for the gaseous medium with an exterior membrane (101), wherein the exterior membrane (101) is made of an elastomeric material, so that a shape and an area size of the exterior membrane (101) is adaptable to a pressure of the gaseous medium in the storage volume (Vs), and
mounting a support structure over the exterior membrane (101) in such a way that

a) if a first pressure value in the storage volume (Vs) is reached, a first portion (111) of the exterior membrane (101) contacts the support structure in a force transmitting manner and a second portion (112) of the exterior membrane (101) is contact-free with the support structure, and
b) if a critical pressure value in the storage volume (Vs) is reached, at least one of the support structure and the exterior membrane (101) fails, wherein if the critical pressure value in storage volume (Vs) is reached, the second portion (112) is expandable such that a contribution (Va) to the storage volume (Vs) of at least 10% in comparison to the volume of the storage volume (Vs) with the first pressure value is generated.

14. Storage (100) for storing a gaseous medium, the storage (100) comprising
a membrane for at least partially enveloping a storage volume (Vs), and a gas supply unit (105), which is connected to the storage volume (Vs) for supplying supporting gas to the storage volume (Vs), so that a gas pressure in the storage volume (Vs) is prevented from falling below a predetermined nominal gas pressure and/or from falling below a predefined position limit.

15. Use of a covering as set forth in one of the claims 1 to 11 in a bio gas storage.

Fig. 1

Fig. 2

Fig. 3

EP 2 502 478 A1

Fig. 4

Fig. 5

Fig. 6

EP 2 502 478 A1

Fig. 7

Fig. 8

Fig. 9

Fig. 10 (Prior Art)

Fig. 11 (Prior Art)

Fig. 12 (Prior Art)

Europäisches
Patentamt

European
Patent Office

Office européen
des brevets

# EUROPEAN SEARCH REPORT

Application Number

EP 11 15 9655

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 4 902 304 A (HALLEN WALTER R [US]) 20 February 1990 (1990-02-20) * column 2, line 53 - column 3, line 15 * * column 3, lines 45-50 * * column 4, lines 4-11 * ----- | 1-13,15 | INV. A01C3/02 B65D88/16 F17B1/26 |
| X | DE 20 2007 007060 U1 (SATTLER AG [AT]) 26 July 2007 (2007-07-26) * paragraphs [0033], [0039], [0048] * ----- | 1 | |
| X | WO 2010/136885 A1 (ECOMEMBRANE SRL [IT]; SPEDINI LUIGI [IT]; SPEDINI LORENZO [IT]) 2 December 2010 (2010-12-02) * page 11, paragraph 6 - page 12, paragraph 2 * ----- | 1 | |
| X | EP 1 338 843 A2 (ECOMENBRANE S R L [IT] ECOMEMBRANE S R L [IT]) 27 August 2003 (2003-08-27) * paragraphs [0051] - [0055] * ----- | 1 | |
| X | AT 388 158 B (SATTLER TEXTILWERKE SATTLER TEXTILWERKE [ST]) 10 May 1989 (1989-05-10) * page 3, lines 9-32 * ----- | 1 | TECHNICAL FIELDS SEARCHED (IPC) A01C B65D F17B |

The present search report has been drawn up for all claims

| Place of search | Date of completion of the search | Examiner |
|---|---|---|
| Munich | 27 May 2011 | Ott, Thomas |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

........................................................................

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 11 15 9655

This annex lists the patent family members relating to the patent documents cited in the above-mentioned European search report.
The members are as contained in the European Patent Office EDP file on
The European Patent Office is in no way liable for these particulars which are merely given for the purpose of information.

27-05-2011

| Patent document cited in search report | | Publication date | Patent family member(s) | | Publication date |
|---|---|---|---|---|---|
| US 4902304 | A | 20-02-1990 | NONE | | |
| DE 202007007060 | U1 | 26-07-2007 | AT | 505174 A1 | 15-11-2008 |
| | | | AT | 479871 T | 15-09-2010 |
| | | | WO | 2008128265 A1 | 30-10-2008 |
| | | | EP | 2149005 A1 | 03-02-2010 |
| WO 2010136885 | A1 | 02-12-2010 | NONE | | |
| EP 1338843 | A2 | 27-08-2003 | AT | 473391 T | 15-07-2010 |
| | | | IT | CR20020001 A1 | 21-08-2003 |
| AT 388158 | B | 10-05-1989 | NONE | | |

EPO FORM P0459

For more details about this annex : see Official Journal of the European Patent Office, No. 12/82